# EUROPEAN PATENT APPLICATION

(11) **EP 2 343 098 A1**
(43) Date of publication of application: **13.07.2011**
(21) Application number: 11150487.4
(22) Date of filing: 10.01.2011
(51) Int. Cl.: A61M 25/01, A61M 25/00, A61B 8/08

(54) **Ultrasound guided echogenic catheter and related methods**

(30) Priority: 12.01.2010 US 335771 P
(71) Applicant: Custom Medical Applications, Inc., Johnstown, NY 12095 (US)
(72) Inventor: Racz, Sandor N., Coppell, TX 75019 (US)
(74) Representative: Jansen, Cornelis Marinus

(57) **Abstract**

Described are methods and apparatus for ultrasound guidance of a tool (10) for a clinical procedure performed upon a subject, wherein the improvement includes a catheter (10) having an echogenic structure (30,230,330) disposed in a distal portion of the catheter, which increases the echogenicity of the catheter.

## Description

### PRIORITY CLAIM

This application claims the benefit of U.S. Provisional Patent Application Serial No. 61/335,771, filed January 12, 2010, and titled "ULTRASOUND GUIDED ECHOGENIC CATHETER AND RELATED METHODS."

### TECHNICAL FIELD

The invention relates generally to the field of medical devices and methods. In particular, the invention relates to methods for utilizing ultrasound to guide a particularly designed catheter that has improved echogenic properties, methods of forming catheters having improved echogenic properties, and catheters having improved echogenic properties.

### BACKGROUND

As described in U. S. Patent 6,019,724 to Gronningsaeter et al. (Feb. 1, 2000), the contents of the entirety of which are incorporated herein by this reference, ultrasound can provide useful feedback to a clinician during a clinical procedure.

With the advent of ever more sophisticated ultrasound equipment (*e.g*., a SONOSITE® NANOMAXX®, S Series, MICROMAXX®, or M-TURBO® device, details available on the internet at sonosite.com) having ever improved resolution, the ability of a clinician to optimally place and use a tool, such as a cutting or resecting device, coagulating device, stapler, biopsy forceps, needle, cannula, *etc.* increases.

For example, improper placement of a catheter for delivering anesthesia (*e.g.,* an epidural catheter) can result in 20% to 40% secondary block failure. *See, e.g.,* Tui & Bhargava, Atlas of Ultrasound and Nerve Stimulation-Guided Regional Anesthesia, 16.1 (2007).

Unfortunately, however, with the utilization of tools in the tightest crevices of the body nearby vital organs and tissues, comes the added risk associated with guiding and positioning the tools within the body and the added risk associated with unexpected tool failure.

### DISCLOSURE

Described is an improvement in a method of placement of a small diameter catheter (*e.g*., an epidural catheter) for delivering fluids to and/or sampling fluids from a subject's body cavity or tissue, the improvement comprising utilizing ultrasound guidance to do so. The method is particularly useful for needle and catheter placement for peripheral nerve blocks in pain management.

Also described is an improved small diameter catheter used in the improved method. The improved catheter utilizes a flexible catheter tube that has been modified at a distal portion thereof. Such modifications include incorporating an echogenic structure (*e.g*., a coiled member or spring, an uneven surface, a braided mesh, a cord, *etc.*) at the distal portion, which increases the catheter tube's echogenicity (for improved ultrasound guidance) and, in some embodiments, may strengthen the catheter tube by increasing its break strength and its rigidity (again, for improved guidance by the ultrasound sensor). Where implemented, the reinforced portion of the tube remains flexible, but guidable by ultrasound.

In additional embodiments, the echogenic structure may extend along an aperture formed in the distal portion of the catheter tube.

In yet additional embodiments, the distal end of the catheter tube may be truncated and a portion of the echogenic structure may be substantially flush with the distal end of the catheter tube to enhance ultrasonic imaging.

In yet additional embodiments, the catheter has a cord running therethrough to provide a means for removing broken portions of the catheter from the subject during use. The cord may terminate in a ball or spherical member useful for removing broken portions thereof from the subject.

In certain embodiments, the, for example, metal ball at the catheter's distal end can be used to "stim" or stimulate as is the case with a stimulating catheter.

In yet additional embodiments, the echogenic structure may comprise a spring disposed in a distal portion of the catheter.

In yet additional embodiments, the echogenic structure may comprise a cord having an overlapping portion disposed in the catheter.

Also disclosed are methods of making and utilizing a catheter according to the invention.

### BRIEF DESCRIPTION OF THE DRAWINGS

FIG. 1 depicts an embodiment of the invention associated with a syringe.
FIG. 2 depicts the embodiment of the preceding figure with a portion of a connector hub shown in cross-section.
FIG. 3 depicts an enlarged, partial cross-sectional view of a distal portion of the catheter of the embodiment of the preceding two figures.
FIGs. 4A and 4B each depict an enlarged, partial cross-sectional view of the proximal portion of the catheter in accordance with embodiments of the invention.
FIG. 5 depicts an enlarged, partial cross-sectional view of a distal portion of the catheter including a spring in accordance with another embodiment of the invention.
FIG. 6 depicts an enlarged, partial cross-sectional view of a distal portion of the catheter including an uneven surface in accordance with yet another embodiment of the invention.
FIG. 7 depicts an enlarged, partial cross-sectional view of a distal portion of the catheter including a braided mesh in accordance with yet another embodiment of the invention.
FIG. 8 depicts an enlarged, partial cross-sectional view of a distal portion of the catheter including an overlapping cord in accordance with yet another embodiment of the invention.
FIG. 9 depicts an enlarged, cross-sectional view of an alternative embodiment of the proximal portion of a catheter tube.
FIG. 10 is an enlarged, cross-sectional view of an embodiment of the preceding figure taken along section line 10--10 of the preceding figure. FIG. 11 is a cross-sectional view of an anesthesia assembly.
FIG. 12 is another cross-sectional view of an anesthesia assembly.

### MODE(S) FOR CARRYING OUT THE INVENTION

Illustrations presented herein are not meant to be actual views of any particular catheter or clinical procedure tool, but are merely idealized representations, which are employed to describe embodiments of the invention. Additionally, elements common between figures may retain the same numerical designation.

As described in U.S. Patent 5,490,845 to Racz (Feb. 13, 1996), the entire disclosure of which is incorporated herein by this reference, "Small diameter catheters are used to introduce medication into the spinal canal, spinal space, epidural space, blood vessels, body cavities and the like. Due to their uni-wall construction when undergoing repetitive movement while being subjected to body heat, such small diameter catheters have a tendency to migrate to other body cavities or to kink thereby preventing the flow of medication therethrough. Such problems can be particularly troublesome when a catheter is used within the spinal canal. In the event of migration of the catheter any kinking of the catheter will preclude aspiration and seeing evidence of such migration due to the closure of the lumen of the catheter and the attendant inability to withdraw blood or spinal fluid. Typical prior art catheter placement units for small diameter catheters are shown in U.S. Patent Nos. 3,856,009; 4,518,383; 4,650,472; 5,084,022; 5,106,376; 5,129,889; 5,213,578; and 5,232,442 (the contents of each of which are hereby incorporated herein by this reference). Another problem associated with the use of such small diameter catheters is their susceptibility to breaking and, possibly, leaving portions thereof remaining in a body cavity. Removal of such broken portions of the catheter may be difficult or impossible."

These problems were addressed in U.S. Patent 5,490,845 by preventing occlusion of the catheter to allow the flow of fluids therethrough and allowing the removal of broken portions thereof from the spinal canal, spinal space, epidural space, blood vessels, body cavities and the like during use. Specifically, as described by U.S. Patent 5,899,891 to Racz (May 4, 1999), the contents of which are also incorporated herein by this reference, U.S. Patent 5,490,845 disclosed a flexible catheter which includes a catheter tube containing an intraluminal cord member (cord) extending along the tube's length and protruding out of the tube's distal and proximal ends. The thus placed cord helps to prevent collapse of the tube during fluid administration, and the portion of the cord extending out of the tube's distal end also aids in the retention and removal of parts of the tube, which might break off during use of the catheter.

The incorporated U.S. Patent 5,899,891 goes on to describe "catheters which utilize a flexible tube that has been modified at either one end or both ends. Such modifications generally involve strengthening the interior of a tube end by increasing its break strength and possibly its rigidity, but do not generally involve decreasing the tip's flexibility. In one embodiment, the tube end modifications involve special placement of an intraluminal cord contained within the tube."

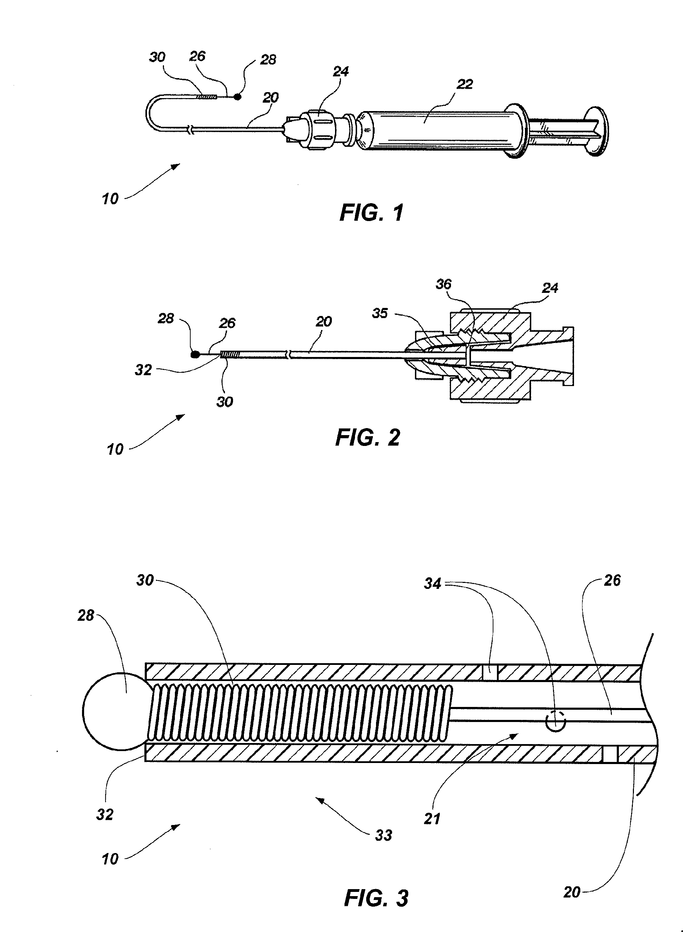

The invention utilizes, in the method and apparatus, catheters that have been modified on a distal portion of the catheter (*e.g*., the catheter tip). Thus, the invention includes a catheter wherein a distal portion of the catheter tube has been modified in such a way as to be echogenic and enhances the ability to create an echo *(i.e.,* return a signal in ultrasound examinations). As depicted in FIGs. 1 and 2, such a modification involves incorporating, within and at the distal portion of the catheter tube, an echogenic structure (*e.g*., a coiled member or spring, an uneven surface, a braided mesh, a cord, *etc*.) placed at least partially within the tube. In some embodiments, the echogenic structure placed within the catheter tube may also act to support and reinforce the distal portion of the catheter tube in such a manner that the reinforced portion of the catheter tube remains flexible. In some embodiments, the catheter may include a cord running therethrough. The cord may be attached at the distal end of the catheter to a ball or spherical member, to the echogenic structure, or to the catheter tube.

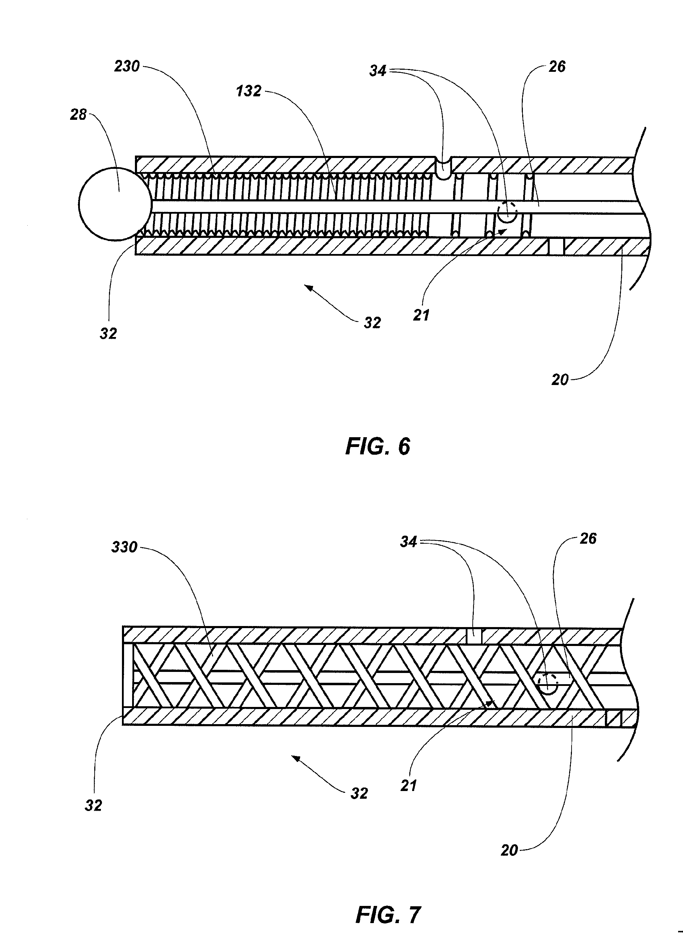

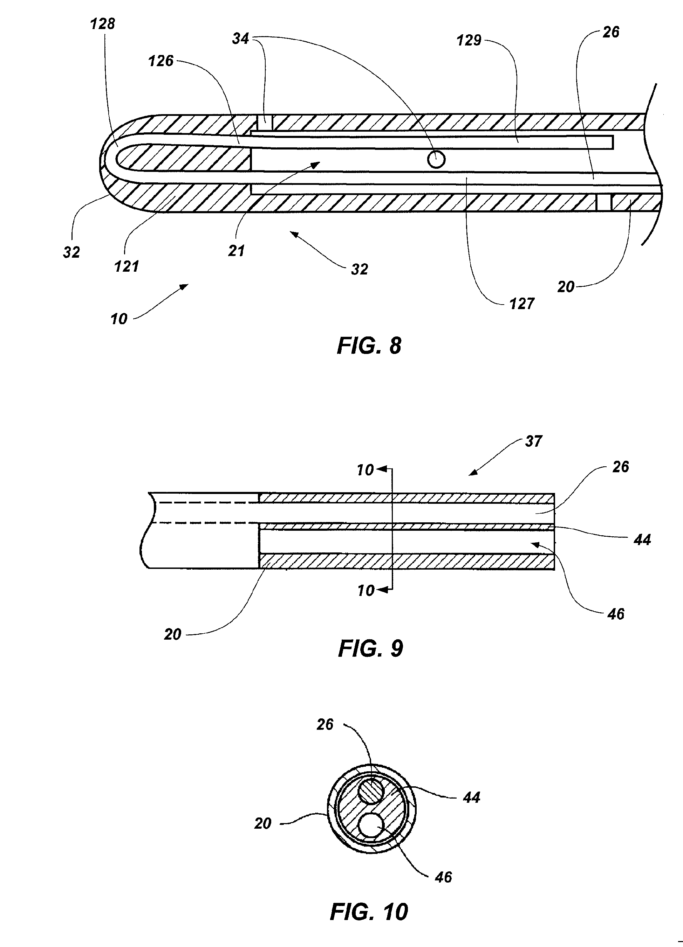

Unlike FIGs. 7 and 8 of U.S. Patent 5,490,521 to Davis et al. (Feb. 13, 1996), the contents of the entirety of which are incorporated herein by this reference, the echogenic structure used in some embodiments of the instant invention may extend all the way to the truncated, flat faced end of the distal tip of the catheter or as nearly as possible to the truncated, flat faced end of the distal tip. Such placement enhances ultrasound imaging, may improve structural integrity of the catheter, and may enhance interaction between the ball and/or spherical member, where implemented, and the distal catheter tip. In some embodiments, the echogenic structure may enable fluid to pass therethrough in order to fluidly couple the lumen of the catheter tube with holes formed in the sidewalls of the catheter tube. In additional embodiments, the echogenic structure may comprise an overlapped cord which may provide enhance ultrasound imaging and may aid in the retention and removal of parts of the catheter tube which might break off during use of the catheter.

The thus placed echogenic structure acts to increase the echogenicity of the catheter tip for better detection by ultrasound, to increase the rigidity of the distal portion of the catheter (while maintaining flexibility). Where the echogenic structure is implemented with a cord and ball structure, the echogenic structure may increase the rigidity of the distal portion of the catheter still interacting with the ball if need be to collect a portion of the tube which may break off the remainder of the tube.

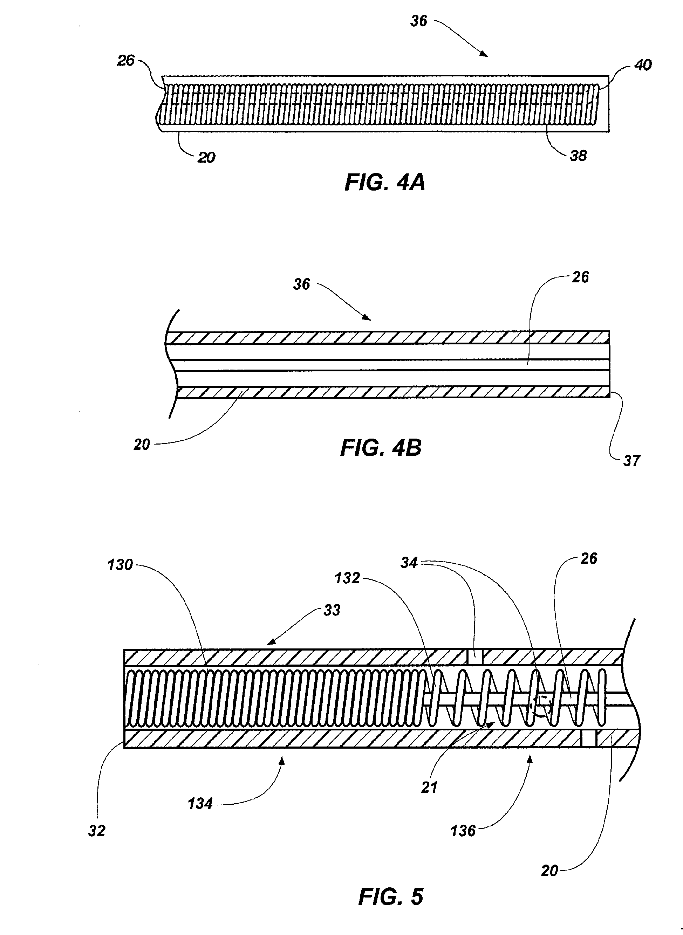

As shown in FIGS. 1 and 2, a catheter 10 may be used in conjunction with, for example, a syringe 22 having a connector hub 24 thereon. As shown, the catheter 10 includes a hollow cylindrical member such as a catheter tube 20 having an intraluminal member (*e.g*., a cord 26) extending therethrough and terminating at the distal end 32 of the catheter tube 20. In some embodiments, the cord 20 may terminate at an enlarged end 28 (*e.g*., at a ball or spherical member) of the catheter tube 20. In other embodiments (*e.g*., the embodiment shown and described with reference to FIG. 5), the cord 20 may terminate at the spring 30 located proximate to the distal end 32 of the catheter tube 20. The tube 20 may have a length from about twenty-five (25) centimeters (10 inches) to about ninety (90) centimeters (thirty-six inches). As shown in FIGs. 1 and 2, the distal end 32 the catheter tube 20 may be substantially truncated with a flat face. The end of the cord 26 may extend from the lumen 21 of the catheter tube 20, and be free to move within the lumen 21 of the catheter tube 20.

The cord 26 for use with catheters such as those disclosed herein may include a cable having several wound metal wires (made of, for example, stainless steel). The cord 26 can also be a braided line including at least three optionally braided cables. Such cords are strong, hypoallergenic, and flexible. Alternatively, the cord 26 may be made of a metal wire, other electrically conductive material, plastic (*e.g*., nylon), other polymers, silk, or other suitable material. In some embodiments, the cord 26 may be manufactured to contain anti-thrombogenic agents or other materials, so as to prevent, for example, occlusion of the catheter during longer term use. In additional embodiments, other chemical agents may be introduced such as, for example, antiseptics and anesthetics.

Referring to FIG. 2, the connector hub 24 is shown in conjunction with the catheter tube 20. The catheter tube 20 is retained within connector hub 24 by interaction of bushing 35 with the proximal portion 36 of the catheter tube 20. In the event the cord 26 is not somehow affixed to the proximal portion 36 of the catheter tube 20, the cord 26 may be trapped between the bushing 35 and the male portion of the connector hub 24.

FIG. 3 depicts an enlarged, partial cross-sectional view of the distal portion 33 of the catheter 10 shown in FIGs. 1 and 2. Referring to FIG. 3, an echogenic structure may be placed at least partially within the catheter tube 20. For example, a coiled member such as a spring 30 may be placed at least partially within the catheter tube 20 proximate to the distal end 32 of the catheter tube 20. The exterior cross-sectional diameter of the spring 30 approximates that of the cross-sectional diameter of the lumen 21 of the catheter 10. The cord 26 may extend through the spring 30. In some embodiments, the spring 30 may extend all the way to the end of the catheter tube 20 and may be substantially flush with the end of catheter tube 20. The spring 30 may be glued, heat formed or melted to the catheter tube 20. A plurality of apertures or holes 34 (*e.g*., holes about 0.4 millimeters ("mm") (or about 0.015 inches) in diameter) may be formed in the sidewall of the catheter 10 at the distal portion 33 thereof so as to enable the fluid to be transported by the catheter 10 to pass therethrough. Should the enlarged end 28 block the lumen 21 at the distal end 32 of the catheter tube 20, the plurality of holes 34 in the distal portion 33 may enable fluid to pass from the lumen 21 of the catheter tube 20 to the holes 34 formed in the sidewall of the catheter tube 20 and from the holes 34 to the lumen 21 (*e.g*., to delivering fluids to and/or sampling fluids from a subject's body cavity or tissue). As also shown in FIG. 3, the spring 30 may be located in the distal portion 33 of the catheter tube 20 such that the spring 30 does not interfere with fluid passing through the lumen 21 of the catheter tube 20 and the holes 34 formed in the catheter tube 20.

The spring may be formed from stainless steel, and the pitch of the spring may be adjusted (or the spread in the coil can be adjusted) for increased echogenicity.

In some embodiments, the catheter tube 20, the cord 26, and the spring 30 may be melted together. The length of the thus formed solid portion is chosen (e.g., from about one-half to about twice the outer diameter of the catheter tube 20) so as not to interfere with the holes 34 formed in the sidewall of the catheter tube 20 while still increasing the strength of the distal portion 33 of the catheter tube 20.

In some embodiments, the cord 26 may be attached (*e.g*., by adhesion, welding, molding or the like) to a portion of the spring 30. For example, the cord 26 may be welded (*e.g*., tack welded) to an interior portion of the spring 30 and may interface with the spring 30 in manner similar to the attachment of the spring 38 and cord 26 shown in FIG. 4A. In some embodiments, the enlarged end 28 of the cord 26 may be attached to the spring 30. For example, a welding process (*e.g*., a tungsten inert gas welding process) may be used to form the enlarged end 28 of the cord 26 and to attach to the cord 26 to the spring 30.

FIG. 4A depicts an enlarged, partial cross-sectional view of the proximal portion 36 of the catheter tube 20 shown in FIGs. 1 and 2. As depicted in FIG. 4A, the cord 26 may be associated (*e.g*., by adhesion, welding, molding or the like) to a coiled member such as, for example, a spring 38 contained within the proximal portion 36 of the catheter tube 20 at weld 40. The spring 38 may be attached to the catheter tube 20 with adhesive placed along the length or around the circumference of the spring 38. In some embodiments, the cord 26 may be adhered to the spring 38 by the use of an adhesive. The coiled member 38 defines a lumen through which fluid flows (and thus also through the lumen 21 of the tube 20) while still increasing the strength of the proximal portion 36 of the catheter tube 20.

FIG. 4B depicts an enlarged, partial cross-sectional view of the proximal portion 36 of the catheter tube 20 in accordance with another embodiment of the invention. As shown in FIG. 4B, the cord 26 may be free-floating (*e.g*., not attached to the catheter tube 20) at the proximal portion 36 of the catheter tube 20. In some embodiments, the cord 26 may be cut to be substantially flush (or slightly recessed) with respect to the proximal end 37 of the catheter tube 20. In such an embodiment, the cord 26 may be coupled to an additional structure such as, for example, the connector hub 24 (FIG. 2).

FIG. 5 depicts an enlarged, partial cross-sectional view of a distal portion 33 of the catheter tube 20 in accordance with another embodiment of the invention. Referring to FIG. 5, an echogenic structure such as a spring 130 may be placed at least partially within the catheter tube 20 proximate to the distal end 32 of the catheter tube 20. The exterior cross-sectional diameter of the spring 130 approximates that of the cross-sectional diameter of the lumen 21 of the catheter tube 20. The spring 130 may be located at the distal portion 33 of the catheter tube 20 such that the spring 130 does not interfere with the fluid coupling of the lumen 21 of the catheter tube 20 with the holes 34 formed in the sidewall of the catheter tube 20. For example, a portion of the spring 130 may contain a pitch (*i.e*., the distance from the center of one coil to the center of an adjacent coil) that enables fluid to flow between the coils 132 of the spring 130 and exit the lumen 21 of the catheter tube 20 through the holes 34. In some embodiments, the spring 130 may include a first portion 134 having a relatively small pitch and a second portion 136 having a relatively larger pitch (*i.e.,* the second portion 136 has a pitch greater than the first portion 134). As shown in FIG. 5, the first portion 134 of the spring 130 may be similar to the spring 30 or a portion thereof shown and described with reference to FIG. 3. The relatively larger pitch of the second portion 136 may enable fluid communication *(i.e.,* fluid coupling) between the lumen 21 of the catheter tube 20 and the holes 34 formed in the catheter tube 20.

FIG. 6 depicts an enlarged view of a distal portion 33 of the catheter tube 20 in accordance with another embodiment of the invention. Referring to FIG. 6, an echogenic structure such as a structure having an uneven surface 230 (*e.g*., illustrated as a series of ridges similar to a coiled member) may be located within the catheter tube 20 proximate to the distal end 32 of the catheter tube 20. The uneven surface 230 may be formed on the inner surface of the catheter tube 20 that defines the lumen 21. In some embodiments, the uneven surface 230 may be a structure formed separate from the catheter tube 20 and disposed therein proximate to the distal end 32 of the catheter tube 20. The uneven surface 230 may be uneven in the fact that if the inner surface of the catheter tube 20 were to be laid flat, a portion of the inner surface (*e.g*., the uneven surface 230) would be non-planar. The uneven surface 230 may be located at the distal portion 33 of the catheter tube 20 such that the structure forming the uneven surface 230 does not interfere with fluid passing through the lumen 21 of the catheter tube 20 and the holes 34 formed in the catheter tube 20. For example, openings in the structure of the uneven surface 230 proximate to the holes 34 formed in the catheter tube 20 may enable fluid communication between the lumen 21 of the catheter tube 20 and the holes 34 formed in the sidewall of the catheter tube 20.

FIG. 7 depicts an enlarged view of the distal portion 33 of the catheter tube 20 in accordance with another embodiment of the invention. Referring to FIG. 7, an echogenic structure such as a braided mesh 330 may be placed at least partially within the catheter tube 20 proximate to the distal end 32 of the catheter tube 20. The exterior cross-sectional diameter of the braided mesh 330 approximates that of the cross-sectional diameter of the lumen 21 of the catheter tube 20. The braided mesh 330 may be located at the distal portion 33 of the catheter tube 20 such that the braided mesh 330 does not interfere with fluid passing through the lumen 21 of the catheter tube 20 and the holes 34 formed in the catheter tube 20. For example, the braided mesh 330 may contain openings formed therein (*e.g*., openings formed between overlapping portions of the braided mesh 330) that enable fluid to flow through the braided mesh 330 and may enable fluid communication between the lumen 21 of the catheter tube 20 and the holes 34 formed in the sidewall of the catheter tube 20.

FIG. 8 depicts an enlarged view of the distal portion 33 of the catheter tube 20 in accordance with another embodiment of the invention. Referring to FIG. 8, the catheter tube 20 includes an echogenic structure such as a cord 126 (*e.g*., a braided cord) extending therethrough. Similar to the cord 26 shown and described with reference to FIGs. 1 through 4B, the cord 126 includes a first portion 127 that at least partially extends from a proximal portion of the catheter tube 20 to a distal portion 33 of the catheter tube 20. For example, the cord 126 may extend from the distal portion 33 of the catheter tube 20 to a proximal portion 36 of the catheter tube 20 and may be free-floating (*e.g*., not attached to the catheter tube 20) at the proximal portion 36 of the catheter tube 20 as shown in FIG. 4B. The cord 126 may also be secured to the catheter 10 to provide a means for removing broken portions of the catheter 10 from the subject during use.

Referring still to FIG. 8, the cord 126 also includes a folded-over portion 128 at the distal portion 33 of the catheter tube 33. The folded over portion 128 enables a second portion 129 of the cord 126 to extend from the distal portion 33 of the catheter tube 20 back toward the proximal portion of the catheter tube 20 and forms an overlapping section of the cord 126 *(i.e.,* the second portion 129 partially overlaps the first portion 127 of the cord 126 along a lateral axis of the cord 126). In some embodiments, the second portion 129 of the cord 126 may extend (in a direction toward the proximal portion of the catheter tube 20) to the most proximal hole 34 *(i.e.,* the hole 34 being located closest to the proximal portion of the catheter tube 20). In some embodiments, a portion of the folded over portion 128 of the cord 126 may be located proximate to the distal end 32 of the catheter tube 20. For example, the folded over portion 128 of the cord 126 may be formed within a solid end 121 of the catheter tube 20 *(i.e.,* the folded over portion 128 of the cord 126 is substantially surrounded by the material of the catheter tube 20). In such an embodiment, the folded over portion 128 of the cord 126 extends substantially to the distal end 32 of the catheter tube 20 and is covered by the catheter tube 20 at the distal end 32 thereof. In other embodiments, the distal end 32 of the catheter tube 20 may be open to the lumen 21 and the cord may be otherwise secured at the distal end 32 (*e.g*., secured in the sidewall of the catheter tube 20, secured by a ball or spherical member, *etc*.). The cord 126 may form an opening between the first portion 127 and second portion 129 thereof that enables fluid communication between the lumen 21 of the catheter tube 20 and the holes 34 formed in the sidewall of the catheter tube 20.

In some embodiments, an echogenic structure (*e.g*., a spring, uneven surface, braided mesh, cord, *etc.*) may be disposed within a clinical procedural tool (*e.g.,* a catheter) such as a flexible spinal needle at the distal end thereof. Such flexible spinal needles are disclosed in, for example, United States Patent Application Publication US 2008/0065017 A1, which application was filed October 31, 2007 and entitled "Method of Using Flexible Spinal Needle Assemblies," the entire disclosure of which is incorporated herein by this reference. For example, as described in the incorporated US 2008/0065017 A1, a flexible spinal needle assembly may include a catheter such as a flexible needle. The flexible needle may include an echogenic structure such as those described herein within the flexible needle. In some embodiments, the flexible need may include a cord (similar to the cords 26, 126 described herein) comprised of braided wires (*e.g*., 16 count 0.0254 millimeter (0.001 inch) braided wires) forming an echogenic structure at a distal portion of the flexible needle.

FIGs. 9 and 10 depict an alternative embodiment of a proximal portion 36 of a catheter tube 20. In that embodiment, the proximal portion 36 of the tube is modified and strengthened by incorporation of an adhesive 44 in the first one (1) to about four (4) centimeters of the tube. As shown in FIGs. 9 and 10, the adhesive is formed to contain a channel 46 (*e.g*., one having a diameter of about 0.3 mm (0.012 inches)) to allow fluid flow therethrough while securing the cord 26 within the catheter tube 20.

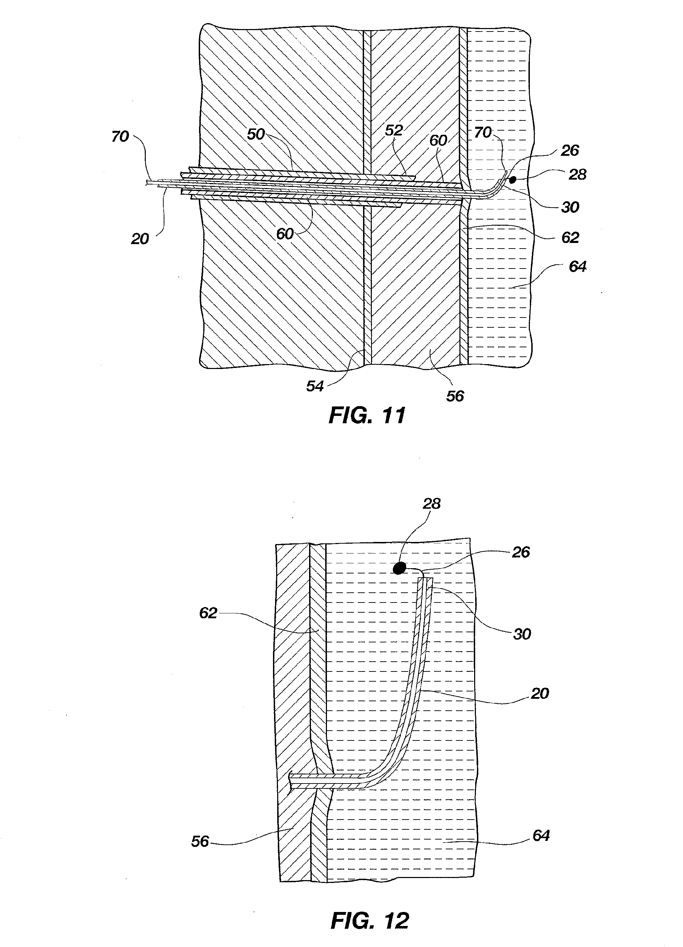

Referring to FIGs. 11 and 12, a catheter tube 20 is shown in use in the spinal canal of a subject (*e.g*., a mammal, such as a human). Under ultrasound guidance (sensor not shown), an epidural needle 50 is inserted at a slight angle to a patient's skin until point 52 passes through ligament 54 and into the epidural space 56. Once the epidural space 56 has been reached by point 52 of needle 50, an introducer 60 is inserted through the lumen of needle 50 until it abuts dura wall 62, which can be imaged by the ultrasound equipment. Subsequently the catheter tube 20 with stylet 70 in place is inserted through the lumen of the introducer 60. The stylet 70 penetrates the dura wall 62 by spreading the fibers thereof. Both the catheter tube 20 and stylet 70 are advanced into the subarachnoid space 64 under ultrasound guidance. Stylet 70 is then removed, and a syringe is used to withdraw spinal fluid. The proximal portion of the catheter tube 20 is maintained outside the patient's body and may be coupled to any desired tubing, syringe, etc. As can be readily seen, if any portion of the catheter tube 20 in the patient is broken, it can be retrieved by pulling on the integrated or non-integrated cord 26. Also if the catheter tube 20 should become kinked, the cord 26 will allow the flow of fluids through the catheter.

As also can been seen in FIGs. 11 and 12, an echogenic structure (*e.g*., the spring 30) located in the distal portion of the catheter tube 20 may enhance the ability of the distal portion of the catheter tube 20 to create an echo (*i.e.,* to be echogenic and return a signal in ultrasound examinations). By increasing the ability of the distal portion of the catheter tube 20 to create an echo, the distal portion of the catheter tube 20 may be relatively more easily identified and located during a procedure utilizing ultrasonic guidance to place a catheter within a subject.

Electrical epidural stimulation of the distal tip can be used to confirm epidural catheter placement in the epidural space in both adult and pediatric patients. Motor responses from electrical stimulation are interpreted particularly in the event of misplacing an epidural catheter (for instance, subarachnoid, subdural, or intravascular). For instance bipolar electrical stimulation can be used in such an embodiment. However, a specialized catheter can provide many other functions, including monitoring of spinal cord potentials, measurement of epidural pressure, and spinal cord stimulation.

Although shown in use as an epidural catheter, the invention may also be used for, *e.g*., intrathecal administration of medicines to a subject.

After being apprised of the devices according to the invention, methods of making them will become readily apparent to those of skill in the art. For instance, a catheter tube may be made of flexible pre-tapered, pre-holed TECOTHANE® 55D polyurethane tubing 0.9 mm (0.035 inch) outer diameter, 0.6 mm (0.025 inch) inner diameter) or nylon (*e.g*., PEBAX® 55D). Alternatively, it can be made of a synthetic absorbable polymer in a manner similar to that disclosed in U.S. Patent 5,129,889 to Hahn et al. (Jul. 14, 1992), the contents of which are incorporated herein by this reference. The cord may be, for example, twisted 0.009 1 X 2 stainless or 0.25 mm (0.010 inch) diameter nylon. The enlarged portion can thus have a diameter of 0.86 mm (0.035 inches). The coiled member may be made of 304 stainless springs (0.6 mm (0.024 inch) outer diameter, 0.4 mm (0.016 inch) inner diameter). The braided mesh and structure including the uneven surface may be made from a metal (*e.g.,* 304 stainless steel), a polymer (*e.g*., nylon), *etc.* The adhesive may be UV cured flexible adhesive (type AAS 2465 LV).

For instance, a process for producing the improved catheter can include obtaining a tube of appropriate length, placing and securing an echogenic structure (*e.g*., a spring) within a distal portion of the tube, and cutting through a distal end of the tube (*e.g*., with a razor under sterile conditions) so that a portion of the spring is substantially flush with the distal end of the tube.

The invention includes a method of forming a tool for a clinical procedure performed upon a subject utilizing ultrasound guidance, the method comprising: forming a catheter having a lumen formed therein; disposing an echogenic structure within the lumen of the catheter; and locating a portion of the echogenic structure at a distal end of the catheter to improve the echogenicity of the distal end of the catheter.

In such an embodiment an echogenic structure may be disposed within the lumen of the catheter comprising disposing a spring within the lumen of the catheter and locating a portion of the spring flush with the distal end of the catheter.

Such an embodiment can further comprise forming a portion of the spring to exhibit a pitch that enables fluid to flow between at least two coils of the spring.

In such embodiments, an echogenic structure may be disposed within the lumen of the catheter comprises disposing a braided mesh within the lumen of the catheter.

In such embodiments, an echogenic structure may be disposed within the lumen of the catheter further comprises forming an uneven surface within the lumen of the catheter.

In such embodiments, an echogenic structure may be disposed within the lumen of the catheter further comprising: disposing a cord within the lumen of the catheter; and folding the cord such that a first portion of the cord extends from a proximal portion of the catheter to a distal portion of the catheter and a second portion of the cord extends from the distal portion of the catheter toward the proximal portion of the catheter. In such an embodiment, the cord may be secured to the distal end of the catheter to provide a means for removing broken portions of the catheter from the subject during use.

The invention also includes a method for ultrasound guidance of a tool for a clinical procedure performed upon a subject, wherein the improvement comprises: utilizing, as the tool, a catheter having an echogenic structure at a distal portion thereof for improved echogenicity, together with means for removing broken portions of the catheter from the subject during use. Such an embodiment may, further comprise forming the echogenic structure and the means for removing broken portions of the catheter from a single cord.

Such an embodiment may further comprise overlapping a portion of the cord at a distal end of the catheter to form the echogenic structure.

Such embodiments may further comprise forming the echogenic structure from a spring; and extending a portion of the spring to a distal end of the catheter. In such an embodiment, the means for removing broken portions of the catheter may comprise extending a cord through the catheter and terminating the cord at the distal end of the catheter with a ball or spherical member. Such an embodiment may further comprise abutting a portion of the spring, when the catheter is being withdrawn from the subject, with the ball or spherical member.

Such embodiments may further comprise forming the cord to increase echogenicity thereof.

Such embodiments may further comprise increasing echogenicity of the distal end of the catheter for improved imaging by an ultrasound sensor used to guide placement of the catheter with the spring and the ball or spherical member.

The invention also includes a catheter for delivering fluids to and/or sampling fluids from a body cavity or tissue of a subject with the aid of ultrasound guidance, the catheter comprising: a catheter tube having a lumen formed therein, the catheter tube having a plurality of apertures formed in a sidewall of the catheter tube at a distal portion thereof; and an echogenic structure at least partially disposed within the distal portion of the catheter tube, the echogenic structure extending along at least one aperture of the plurality of apertures formed in a sidewall of the catheter tube and increasing echogenicity of the distal portion of the catheter for improved imaging by an ultrasound sensor used to guide placement of the catheter.

In such a catheter, the echogenic structure may form at least one opening therein, the at least one opening enabling the lumen formed in the catheter tube to be in fluid communication with at least one aperture of the plurality of apertures formed in a sidewall of the catheter tube.

In such an embodiment, the echogenic structure may comprise a spring including a plurality of coils, at least two coils of the plurality of coils having a pitch that forms the at least one opening in the echogenic structure.

In such an embodiment, the spring may comprise a first portion having a first pitch and a second portion having a second pitch, wherein the first pitch is less than the second pitch and wherein a portion the second pitch forms the at least one opening in the echogenic structure.

In such an embodiment, the echogenic structure may comprise a braided mesh, wherein overlapping portions of the braided mesh form the at least one opening in the echogenic structure.

The invention further includes a catheter for delivering fluids to and/or sampling fluids from a body cavity or tissue of a subject with the aid of ultrasound guidance, the catheter comprising: a catheter tube having a proximal end and a distal end, wherein the distal end is a truncated flat faced portion of the catheter tube; and an echogenic structure at least partially disposed within a distal portion of the catheter tube and a portion of the echogenic structure being substantially flush with the distal end of the catheter tube, the echogenic structure increasing echogenicity of the distal portion of the catheter for improved imaging by an ultrasound sensor used to guide placement of the catheter.

In such a catheter, the echogenic structure may comprise a spring.

In such an embodiment, the spring may comprise a first portion having a first pitch and a second portion having a second pitch, the second pitch being greater than the first pitch, and wherein the second pitch enables fluid within the catheter tube to be in fluid communication with at least one aperture formed in a sidewall of the catheter tube.

In such embodiments, the echogenic structure may comprise a braided mesh.

In such embodiments, the echogenic structure may comprise a cord, the cord comprising: a first portion extending from a proximal portion of the catheter tube to a distal portion of the catheter tube; and a second portion extending from the distal portion of the catheter tube toward the proximal portion of the catheter tube, the first portion and the second portion being connected at a folded-over portion, a portion of the folded-over portion being disposed substantially flush with the distal end of the catheter tube.

The invention further includes a catheter for delivering fluids to and/or sampling fluids from a body cavity or tissue of a subject with the aid of ultrasound guidance, the catheter comprising: a catheter tube having a lumen formed therein; and a cord at least partially disposed in the lumen of the catheter tube, the cord comprising: a first portion extending from a proximal portion of the catheter tube to a distal portion of the catheter tube; and a second portion extending from the distal portion of the catheter tube toward the proximal portion of the catheter tube, the first portion and the second portion being connected at a folded over portion.

With such a catheter, the folded over portion may extend substantially to the distal end of the catheter tube and where the folded over portion is substantially surrounded by the catheter tube to form a solid end portion.

The invention further includes a catheter for delivering fluids to and/or sampling fluids from a body cavity or tissue of a subject with the aid of ultrasound guidance, the catheter comprising: a tube having a proximal end and a distal end, wherein the distal end is a truncated flat faced portion of the tube; a spring positioned within a distal portion of the tube, and extending all of the way to the distal end of the tube, for improved echogenicity; and a cord extending through the tube and terminating in a ball or spherical member useful for removing broken portions thereof from the subject; wherein, upon withdrawal of the catheter from the subject during use, the ball or spherical member abuts the spring and the distal end of the catheter, and further wherein the ball or spherical member, together with the spring, increases echogenicity of the distal portion of the catheter for improved imaging by an ultrasound sensor used to guide placement of the catheter.

The invention also includes a process for producing such a catheter, the process comprising: obtaining a tube of appropriate length; placing and securing the spring within the distal portion of the tube; and cutting through the distal portion of the tube so that a portion of the spring is substantially flush with the distal end of the tube.

## Claims

1. A method of forming a tool for a clinical procedure performed upon a subject utilizing ultrasound guidance, comprising:
forming a catheter having a lumen formed therein;
disposing an echogenic structure within the lumen of the catheter; and
locating a portion of the echogenic structure at a distal end of the catheter to improve the echogenicity of the distal end of the catheter.

2. The method according to claim 1, wherein disposing an echogenic structure within the lumen of the catheter comprises disposing a spring within the lumen of the catheter and locating a portion of the spring flush with the distal end of the catheter.

3. The method according to claim 2, further comprising cutting through the distal portion of the tube so that a portion of the spring is substantially flush with the distal end of the tube.

4. The method according to claim 2 or 3, further comprising forming a portion of the spring to exhibit a pitch that enables fluid to flow between at least two coils of the spring.

5. The method according to claim 1, wherein disposing an echogenic structure within the lumen of the catheter further comprises:
disposing a cord within the lumen of the catheter; and
folding the cord such that a first portion of the cord extends from a proximal portion of the catheter to a distal portion of the catheter and a second portion of the cord extends from the distal portion of the catheter toward the proximal portion of the catheter.

6. The method according to any one of claims 1 through 5, further comprising forming the echogenic structure within the lumen of the catheter to provide a means for removing broken portions of the catheter from the subject during use.

7. The method according to claim 6, wherein forming the echogenic structure within the lumen of the catheter to provide a means for removing broken portions of the catheter from the subject during use comprises extending a cord through the catheter and terminating the cord at the distal end of the catheter with a ball or spherical member.

8. The method according to claim 7, further comprising increasing echogenicity of the distal end of the catheter for improved imaging by an ultrasound sensor used to guide placement of the catheter with at least one of the cord, the spring, and the ball or spherical member.

9. A catheter for delivering fluids to and/or sampling fluids from a body cavity or tissue of a subject with the aid of ultrasound guidance, the catheter comprising:
a catheter tube having a lumen formed therein, the catheter tube having a plurality of apertures formed in a sidewall of the catheter tube at a distal portion thereof; and
an echogenic structure at least partially disposed within the distal portion of the catheter tube, the echogenic structure extending along at least one aperture of the plurality of apertures formed in a sidewall of the catheter tube and increasing echogenicity of the distal portion of the catheter for improved imaging by an ultrasound sensor used to guide placement of the catheter.

10. The catheter according to claim 9, wherein the echogenic structure forms at least one opening therein, the at least one opening enabling the lumen formed in the catheter tube to be in fluid communication with at least one aperture of the plurality of apertures formed in a sidewall of the catheter tube.

11. The catheter according to claim 10, wherein the echogenic structure comprises a spring including a plurality of coils, at least two coils of the plurality of coils having a pitch that forms the at least one opening in the echogenic structure.

12. The catheter according to claim 10, wherein the echogenic structure comprises a braided mesh, wherein overlapping portions of the braided mesh form the at least one opening in the echogenic structure.

13. The catheter according to any one of claims 10 through 12, wherein the distal end of the catheter tube comprises a truncated flat faced portion of the catheter tube and wherein a distal end of the echogenic structure is substantially flush with the distal end of the catheter tube.

14. The catheter according to claim 10, wherein the echogenic structure comprises a cord, the cord comprising:
a first portion extending from a proximal portion of the catheter tube to the distal portion of the catheter tube; and
a second portion extending from the distal portion of the catheter tube toward the proximal portion of the catheter tube, the first portion and the second portion being connected at a folded-over portion, a portion of the folded-over portion being disposed substantially flush with the distal end of the catheter tube.

15. The catheter according to claim 14, wherein the folded over portion is at least partially surrounded by the catheter tube to form a solid end portion.

16. Use of a tool according to any one of claims 1 through 15 for ultrasound guidance for a procedure performed upon a subject.
